# EUROPEAN PATENT APPLICATION

(11) **EP 4 039 794 A1**
(43) Date of publication of application: **10.08.2022**
(21) Application number: 20870648.1
(22) Date of filing: 29.09.2020
(51) Int. Cl.: C12N 1/20, A61K 35/74, A61P 31/04, A61P 37/04, C12R 1/45

(54) **STAPHYLOCOCCUS EPIDERMIDIS STRAIN HAVING IMMUNITY-ENHANCING ACTIVITY AND ANTIBACTERIAL ACTIVITY AGAINST PATHOGENIC BACTERIA, AND USE THEREOF**

(30) Priority: 02.10.2019 KR 20190122293
(71) Applicant: Biome Inc., Seodaemun-gu Seoul 03722 (KR)
(72) Inventor: YOON, Sang Sun, Seoul 03477 (KR); KIM, Gwang Hee, Chuncheon-si Gangwon-do 24209 (KR); LEE, Kang Mu, Seoul 06344 (KR)
(74) Representative: Fabry, Bernd
(86) International application number: PCT/KR2020/013433
(87) International publication number: WO 2021/066585

(57) **Abstract**

The present specification provides a *Staphylococcus epidermidis* strain deposited under accession no. KCTC13941BP and having immunity-enhancing activity.

## Description

### [Technical Field]

The present invention relates to a *Staphylococcus epidermidis* strain having immune-enhancing activity and antibacterial activity against pathogenic bacteria and uses thereof.

### [Background Art]

Chronic respiratory diseases such as asthma, cystic fibrosis, and chronic obstructive pulmonary disease (COPD) are chronic diseases with a large burden of disease and require continuous preventive management and treatment. As a cause of these respiratory diseases, there is *Pseudomonas aeruginosa,* which is known as an opportunistic human harmful bacterium.

*Pseudomonas aeruginosa* is a gram-negative *Bacillus* widely distributed in natural environments such as soil and water and causes intractable and lethal infections. The main target of *Pseudomonas aeruginosa* is an infectious patient (host) whose biological defense function is weakened. In particular, *Pseudomonas aeruginosa* can be fatal to patients with respiratory diseases in which the microbial excretion function of the mucus layer existing on the bronchi is reduced. Antibiotic administration having anti-*Pseudomonas aeruginosa* activity is used for the treatment of such *Pseudomonas aeruginosa.* However, *Pseudomonas aeruginosa* is known as a strain with multi-resistance. Accordingly, *Pseudomonas aeruginosa* has resistance to antibiotics, so it is difficult to expect an effect of antibiotic prescription for the purpose of cure.

For these reasons, the development of a new therapeutic composition capable of effectively inhibiting pathogenic bacteria including *Pseudomonas aeruginosa* to have a therapeutic effect on infectious diseases caused by pathogens and reducing the use of antibiotics is continuously required.

The background of the present invention is described in order to facilitate understanding of the present invention. It should not be construed as an admission that the matters described in the background of the present invention exist as prior art.

### [Disclosure]

### [Technical Problem]

Meanwhile, the present inventors noted that the interaction between the intestinal host and microorganisms, that is, the intestinal microbiome, is involved in the host's immunological defense mechanism.

Furthermore, the present inventors recognized that in addition to the microbiome of the intestinal environment, the microbiome of the respiratory system may be involved in the host's immunological defense mechanism.

Accordingly, the present inventors paid more attention to the relationship between the onset of an infectious disease caused by *Pseudomonas aeruginosa* and the microbial community of the respiratory system.

More specifically, in order to analyze the inhibition of respiratory system infection by *Pseudomonas aeruginosa* according to the formation of a specific microbial community in the respiratory system, the present inventors observed changes to *Pseudomonas aeruginosa* after transplanting the resident flora commonly found in the respiratory system into the respiratory system of sterile laboratory animals.

As a result, the present inventors were able to confirm that infection by *Pseudomonas aeruginosa* can be inhibited by a specific resident flora among the microbial community of the respiratory system. In other words, it was found that a specific resident flora interacts with the host, i.e., the host's innate immune function is improved, and thus the defense mechanism against *Pseudomonas aeruginosa* infection can be improved.

Accordingly, the present inventors developed a novel strain with the ability to inhibit respiratory system pathogens and could recognize that the strain can be used for the treatment and prevention of pathogen infection and thus pathogen-infected disease.

As a result, the present inventors developed a strain having an immune-enhancing activity and an antibacterial activity against pathogenic bacteria, and a pharmaceutical composition for preventing or treating infectious diseases including the same.

Meanwhile, the present inventors identified a strain having antibacterial activity against pathogenic bacteria, and as a result, it was confirmed that the novel strain was *Staphylococcus epidermidis.*

Accordingly, an object of the present invention is to provide a *Staphylococcus epidermidis* strain having antibacterial activity against pathogenic bacteria and a pharmaceutical composition for preventing or treating pathogenic bacteria-induced diseases including the same.

The problems of the present invention are not limited to the problems mentioned above, and other problems not mentioned are clearly understood by those skilled in the art from the following description.

In order to solve the above problems, the present invention provides a *Staphylococcus epidermidis* strain deposited with accession number KCTC13941BP and having an immune-enhancing activity.

### [Technical Solution]

According to a feature of the present invention, immune-enhancing activity is to increase innate immune cells expressing TNF-α, and the innate immune cells may include at least one selected from the group consisting of dendritic cells, monocytes, neutrophils, eosinophils, basophils, macrophages, and natural killer cells, but it is not limited thereto.

According to another feature of the present invention, the number of innate immune cells improved by the *Staphylococcus epidermidis* strain according to an embodiment of the present invention may be increased by 2 times or more than that of an individual not treated with the *Staphylococcus epidermidis* strain. More specifically, the number of dendritic cells may be increased by 2 times or more than that of an individual not treated with the *Staphylococcus epidermidis* strain, and the number of monocytes may be increased by 12 times or more than that of an individual not treated with the *Staphylococcus epidermidis* strain, and the number of neutrophils may be increased by 6 times or more than that of an individual not treated with the *Staphylococcus epidermidis* strain. Meanwhile, monocytes having 12 times or more increase than the individual not treated with *Staphylococcus epidermidis* strain may be CD11b+^{high} cells.

According to another feature of the present invention, the *Staphylococcus epidermidis* strain according to an embodiment of the present invention may exhibit antibacterial activity against pathogenic bacteria, in which the pathogenic bacteria is *Pseudomonas aeruginosa.* However, the present invention is not limited thereto and may include all of the various pathogenic bacteria that can cause infectious diseases. Furthermore, the pathogenic bacteria described above may have resistance to at least one selected from the group consisting of a carbapenem-based antibacterial agent, a beta-lactam-based antibacterial agent, a penicillin-based antibacterial agent, a cephalosporins-based antibacterial agent, and a monobactam-based antibacterial agent but is not limited thereto.

According to another feature of the present invention, the *Staphylococcus epidermidis* strain according to an embodiment of the present invention may be a strain enhanced compared to the intrinsic activity for protease and mucinase.

In order to solve the problems as described above, the present invention provides a pharmaceutical composition for preventing or treating infectious diseases, the composition including the *Staphylococcus epidermidis* strain as described above or a suspension of the *Staphylococcus epidermidis* strain as described above as an active ingredient.

According to a feature of the present invention, the infectious disease may include at least one selected from the group consisting of tuberculosis, pneumonia, urinary tract infection, wound infection, encephalomeningitis, osteomyelitis, wound infection, entophthalmia, endophthalmitis, liver abscess, pharyngitis, diarrhea, sepsis, sinusitis, rhinitis, otitis media, bacteremia, endocarditis, cholecystitis, and parotitis, but is not limited thereto, and may include all of the various diseases that can be caused by infection with pathogenic bacteria.

According to another feature of the present invention, the pharmaceutical composition for preventing or treating an infectious disease may be in the form of an external preparation or a spray, but is not limited thereto, and it may include all various forms that may contain a pharmaceutical composition for preventing or treating an infectious disease.

In order to solve the above problems, the present invention provides a composition for enhancing immunity, the composition including the *Staphylococcus epidermidis* strain as described above or a suspension of the *Staphylococcus epidermidis* strain as described above as an active ingredient.

Hereinafter, the present invention is described in more detail through Examples. However, since these Examples are only for illustrative purposes of the present invention, the scope of the present invention should not be construed as being limited by these Examples.

The present invention may provide a pharmaceutical composition for preventing or treating infectious diseases and a composition for enhancing immunity, the compositions including *Staphylococcus epidermidis* strain having an immune-enhancing activity, a strain thereof and a suspension of the strain as active ingredients.

Accordingly, the present invention may improve innate immune cells of an individual to have antibacterial activity against pathogenic bacteria, and thus may have preventive and therapeutic effects on infectious diseases.

### [Advantageous Effects]

More specifically, the present invention can survive and proliferate in the respiratory tract for a long time, thereby increasing the duration of the defense mechanism effect against pathogenic bacteria. Furthermore, it can have high viability and proliferation in the nasal cavity, so that it can primarily improve the immune effect against pathogenic bacteria in the respiratory system of an individual.

Further, the present invention does not cause an immune response in an individual, unlike pathogenic bacteria and is not as harmful as PBS, so it has stability without toxicity and harm to an individual and can coexist in an individual.

Further, the present invention can increase the number of innate immune cells of an individual and enhance the secretion activity of their cytokines to provide an improved immune-enhancing activity to an individual.

As a result, the present invention can enhance the expression of neutrophils, which are innate immune cells, thereby further enhancing the secondary acquired immune response so that the immune response of the individual can be more efficiently improved.

The effect according to the present invention is not limited by the contents exemplified above, and more various effects are included in the present specification.

### [Description of Drawings]

FIG. 1 exemplarily illustrates a procedure of a method for preparing a pharmaceutical composition for preventing or treating an infectious disease according to an embodiment of the present invention.
FIG. 2 illustrates the survival rate results of germ free (GF) and specific pathogen free (SPF) mouse models according to *Pseudomonas aeruginosa* infection.
FIGS. 3A and 3B illustrate the selection process of *Staphylococcus epidermidis* strains according to an embodiment of the present invention.
FIG. 4 exemplarily illustrates an experimental procedure for transplantation of a *Staphylococcus epidermidis* strain according to an embodiment of the present invention.
FIGS. 5A to 5C illustrates the results of the colonization ability, the survival rate of the specific pathogen free (SPF) mouse model and the survival rate of pathogenic bacteria according to the transplantation of the *Staphylococcus epidermidis* strain according to an embodiment of the present invention.
FIG. 6 illustrates the survival rate results of the germ free (GF) mouse model according to the transplantation of *Staphylococcus epidermidis* strain according to an embodiment of the present invention.
FIG. 7 illustrates the results for confirming the stability of the *Staphylococcus epidermidis* strain according to an embodiment of the present invention.
FIGS. 8A to 8C illustrate the results of immune cell activity according to the transplantation of the *Staphylococcus epidermidis* strain according to an embodiment of the present invention.
FIGS. 9A and 9B illustrate the results of cytokine secretion activity of immune cells according to the transplantation of the *Staphylococcus epidermidis* strain according to an embodiment of the present invention.
FIGS. 10 to 11C illustrate the results of the antibacterial activity against pathogenic bacteria according to the transplantation of the *Staphylococcus epidermidis* strain according to an embodiment of the present invention.

### [Modes of the Invention]

Advantages and features of the present invention, and a method for achieving the same become apparent with reference to the Examples described below in detail in conjunction with the accompanying drawings. However, the present invention is not limited to the Examples disclosed below, but is embodied in various different forms. Only the present Examples are provided to complete the disclosure of the present invention and to fully inform those of ordinary skill in the art to which the present invention pertains to the scope of the present invention. The present invention is only defined by the scope of the claims.

Hereinafter, terms used in the present specification are described for clarity of description.

As used herein, the term "individual" may refer to a subject capable of having immune-enhancing activity and antibacterial activity against pathogenic bacteria by a *Staphylococcus epidermidis* strain according to an embodiment of the present invention or a pharmaceutical composition for preventing or treating an infectious disease and a composition for enhancing immunity, the compositions including a *Staphylococcus epidermidis* strain or a suspension of *Staphylococcus epidermidis* strain as an active ingredient. It may be used interchangeably with terms such as "host," "subject" and "patient."

As used herein, the term "intrinsic activity" may refer to an active state naturally possessed by microorganisms. Here, "*Staphylococcus epidermidis* enhanced compared to intrinsic activity" may refer to *Staphylococcus epidermidis* having improved activity on a specific molecule compared to the activity on a specific molecule that *Staphylococcus epidermidis* originally have. Accordingly, *Staphylococcus epidermidis* with enhanced intrinsic activity may have an improved specific function by enhancing the activity for a specific molecule.

As used herein, the term "vector" refers to DNA constructs containing DNA sequences operably linked to regulatory sequences capable of controlling expression of a protein in a host cell and other sequences introduced to facilitate genetic manipulation or to optimize expression of a protein. Regulatory sequences may include a promoter for regulating transcription, an operator optionally added to control transcription, an appropriate mRNA ribosome binding site, and sequences regulating the termination of transcription and translation.

Specifically, the vector is a vector in which the promoter is a CAT promoter and may include a plasmid vector, a cosmid vector, a bacteriophage vector, a yeast vector, or a viral vector such as an adenoviral vector, a retroviral vector, and an adeno-associated viral vector. The vector may be in a form integrated into the genome of the host cell.

As used herein, the term "recombination" may be genetic recombination, and genetic recombination may mean splicing gene DNA isolated from a heterogeneous organism or a synthesized gene. "Recombinant vector" may refer to a vector that has been recombined to include the polynucleotide represented by SEQ ID NO: 1.

As used herein, the term "transformation" may refer to a change in the genetic properties of an organism by receiving DNA from the outside. The transformation method may include methods using ribosomes, electroporation, chemicals that increase free DNA uptake, direct DNA injection into host cells, particle gun bombardment, and micro-injection, but is not limited thereto. A wider variety of methods may be used as transformation methods.

As used herein, the term "host cell" may refer to a cell used for viral infection or bacteria used for proliferation of vectors such as plasmids or phage DNA and insert genes.

For example, the host cell may be a host cell transformed with the recombinant vector. Transformable host cells include prokaryotic host cells such as *Bacillus subtilis*, *Streptomyces*, *Pseudomonas*, *Proteus mirabilis*, or *Staphylococcus,* but is not limited thereto. In addition, lower eukaryotic cells such as fungi (e.g., *Aspergillus*), yeast (e.g., *Pichia pastoris*), *Saccharomyces cerevisiae, Schizosaccharomyces*, and *Neurospora crassa* may be used as host cells.

Accordingly, in the present specification, a transformed host cell may be used interchangeably with a transformed microorganism.

Meanwhile, cells derived from higher eukaryotes including insect cells, plant cells, mammals, and the like may be used as host cells. However, the present invention is not limited thereto, and more various cells may be used as host cells transformed with the recombinant vector.

As used herein, the term "expression" may mean that a polypeptide is expressed or produced in a gene encoding an esterase in the transformed host cell.

Isolation and purification mean isolating and purifying a polypeptide as an active ingredient. For example, for the process of isolating and purifying the polypeptide according to an embodiment of the present invention, methods known in the art may be used without limitation, and ion exchange chromatography may be preferably used.

### Pharmaceutical composition for preventing or treating infectious diseases

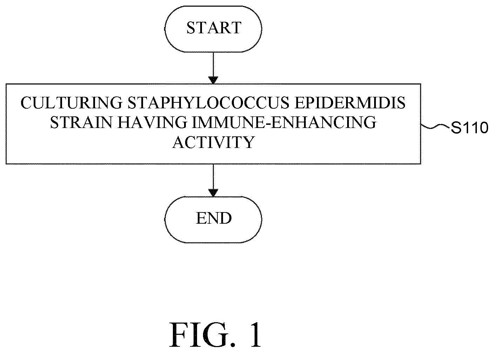

Hereinafter, a pharmaceutical composition for preventing or treating an infectious disease according to an embodiment of the present invention is described with reference to FIG. 1.

First, FIG. 1 illustrates a procedure of a method for preparing a pharmaceutical composition for preventing or treating an infectious disease according to an embodiment of the present invention. The method for preparing a pharmaceutical composition for preventing or treating an infectious disease includes culturing a *Staphylococcus epidermidis* strain having an immune-enhancing activity (S110).

Here, *Staphylococcus epidermidis* is a strain deposited with accession number KCTC13941BP, which is one of the bacteria widely distributed in nature and is a resident flora mainly inhabiting the epithelium such as the skin of the host.

Furthermore, *Staphylococcus epidermidis* used in the pharmaceutical composition for preventing or treating infectious diseases according to an embodiment of the present invention is enhanced compared to the intrinsic activity of protease and mucinase, that is, it can secrete both protease and mucinase. Accordingly, *Staphylococcus epidermidis* used in the pharmaceutical composition for preventing or treating infectious diseases according to an embodiment of the present invention may have excellent colonization ability even in poor environmental conditions because the function of the above-described degrading enzyme is activated.

Furthermore, *Staphylococcus epidermidis* used in the pharmaceutical composition for preventing or treating infectious diseases according to an embodiment of the present invention may have immune-enhancing activity. In other words, *Staphylococcus epidermidis* may increase the host's innate immune cells expressing TNF-α. However, cells expressing TNF-α are not limited to innate immune cells and may include all of the various immune cells whose number can be increased by *Staphylococcus epidermidis* used in the pharmaceutical composition for preventing or treating infectious diseases according to an embodiment of the present invention.

Here, the innate immune cell may mean at least one selected from the group consisting of dendritic cell, monocyte, neutrophils, eosinophils, basophils, macrophage and natural killer cell, and *Staphylococcus epidermidis* used in the pharmaceutical composition for preventing or treating infectious diseases according to an embodiment of the present invention may increase the number of these innate immune cells by 2 times or more than that of an individual not treated with the *Staphylococcus epidermidis* strain.

More specifically, *Staphylococcus epidermidis* used in the pharmaceutical composition for preventing or treating infectious diseases according to an embodiment of the present invention may increase the number of dendritic cells 2 times or more than that of an individual not treated with the *Staphylococcus epidermidis* strain. *Staphylococcus epidermidis* may increase the number of monocytes 12 times or more than that of an individual not treated with the *Staphylococcus epidermidis* strain. *Staphylococcus epidermidis* may increase the number of neutrophils 6 times or more than that of an individual not treated with the *Staphylococcus epidermidis* strain.

Accordingly, *Staphylococcus epidermidis* used in the pharmaceutical composition for preventing or treating infectious diseases according to an embodiment of the present invention may increase innate immune cells, thereby providing an immune effect against various diseases. That is, *Staphylococcus epidermidis* used in the pharmaceutical composition for preventing or treating infectious diseases according to an embodiment of the present invention may increase innate immune cells, thereby exhibiting activity against pathogenic bacteria.

More specifically, *Staphylococcus epidermidis* used in the pharmaceutical composition for preventing or treating infectious diseases according to an embodiment of the present invention may increase innate immune cells, thereby improving antibacterial properties against the pathogenic bacteria, *Pseudomonas aeruginosa.* In other words, *Staphylococcus epidermidis* used in the pharmaceutical composition for preventing or treating infectious diseases according to an embodiment of the present invention may reduce the number of *Pseudomonas aeruginosa* 100 times or less than that of an individual not treated with the *Staphylococcus epidermidis* strain.

Here, the aforementioned pathogenic bacteria may have resistance to antibiotics, that is, antibacterial agents. Such antibacterial agents may include at least one selected from the group consisting of carbapenem-based antibacterial agents, beta-lactam-based antibacterial agents, penicillin-based antibacterial agents, cephalosporins-based antibacterial agents, and monobactams-based antibacterial agents, but is not limited thereto. It may include all of the various antibacterial agents having antibacterial action.

After all, the pharmaceutical composition for preventing or treating infectious diseases according to an embodiment of the present invention may include *Staphylococcus epidermidis* to improve the host's own immunity, thereby overcoming limitations for treatment of pathogenic bacteria with conventional antibiotic (antibacterial agent) resistance.

Furthermore, the pharmaceutical composition for preventing or treating infectious diseases according to an embodiment of the present invention may include *Staphylococcus epidermidis* to exhibit host immunity enhancement and antibacterial activity against pathogenic bacteria, thereby preventing or treating various infectious diseases. Here, the infectious disease may include at least one selected from the group consisting of tuberculosis, pneumonia, urinary tract infection, wound infection, encephalomeningitis, osteomyelitis, wound infection, entophthalmia, endophthalmitis, liver abscess, pharyngitis, diarrhea, sepsis, sinusitis, rhinitis, otitis media, bacteremia, endocarditis, cholecystitis, and parotitis, but is not limited to thereto. It may include all of the various diseases that can be caused by pathogenic bacteria.

Meanwhile, the method for preparing a pharmaceutical composition for preventing or treating an infectious disease according to an embodiment of the present invention may further include the step of formulating after culturing *Staphylococcus epidermidis* strain having immune-enhancing activity (S110). More specifically, when the pharmaceutical composition for preventing or treating infectious diseases according to an embodiment of the present invention is formulated as a liquid solution, it may be diluted with a pharmaceutically acceptable carrier.

More specifically, a pharmaceutically acceptable carrier may refer to a carrier or diluent that does not irritate the organism and does not impair the biological activity and properties of the administered compound. For example, acceptable pharmaceutical carriers for pharmaceutical compositions formulated as liquid solutions are sterile and biocompatible and may be saline, sterile water, Ringer's solution, buffered saline, albumin injection solution, dextrose solution, maltodextrin solution, glycerol, ethanol, and a mixture in which one or more of these components are mixed. Further, other conventional additives such as antioxidants, buffers, and bacteriostats may be added as needed. Furthermore, diluents, dispersants, surfactants, binders and lubricants are additionally added to the pharmaceutical composition for preventing or treating infectious diseases, which may be formulated into injectable formulations such as aqueous solutions, suspensions, emulsions, pills, capsules, granules, or tablets.

The pharmaceutical composition for preventing or treating an infectious disease may be administered orally or parenterally or may be administered by applying or spraying to a diseased site. The pharmaceutical composition for preventing or treating an infectious disease may also be administered parenterally by intravenous administration, intraperitoneal administration, intramuscular administration, subcutaneous administration or local administration.

Appropriate applying, spraying, and dosage of the pharmaceutical composition for preventing or treating infectious diseases may vary depending on factors such as the formulation method, administration method, age, weight, or sex of the target animal and patient, degree of disease symptoms, food, administration time, route of administration, excretion rate and response sensitivity. Further, a skilled doctor or veterinarian can easily determine and prescribe the dosage of a pharmaceutical composition for preventing or treating an infectious disease effective for the desired treatment.

Formulations for oral administration including a pharmaceutical composition for preventing or treating infectious diseases as an active ingredient may be tablets, troches, lozenges, aqueous or oily suspensions, powders, granules, emulsions, hard or soft capsules, syrups or elixirs. Here, in order to formulate into forms such as tablets and capsules, it may include binders such as lactose, saccharose, sorbitol, mannitol, starch, amylopectin, cellulose or gelatin, excipients such as dicalcium phosphate, disintegrants such as corn starch or sweet potato starch, lubricating oils such as magnesium stearate, calcium stearate, sodium stearyl fumarate or polyethylene glycol wax. In the case of capsule formulations, in addition to the above-mentioned substances, a liquid carrier such as fatty oil may further be included.

However, the most preferred formulation and form of the pharmaceutical composition for preventing or treating infectious diseases may be in the form of an external preparation or a spray, but is not limited thereto.

According to the above procedure, the pharmaceutical composition for preventing or treating an infectious disease according to an embodiment of the present invention may have antibacterial activity against pathogenic bacteria by improving the host, that is, the innate immune cells of the individual, thereby having a preventive and therapeutic effect on infectious diseases.

### Confirmation of host susceptibility of symbiotic microorganisms

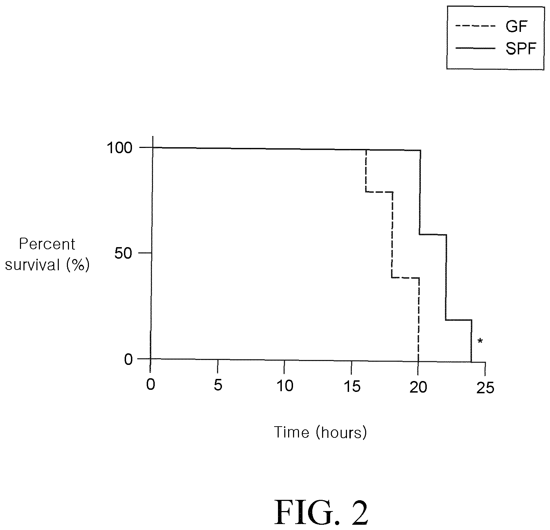

Hereinafter, the host susceptibility of symbiotic microorganisms is described with reference to FIG. 2.

Referring to FIG. 2, the survival rate results of germ free (GF) and specific pathogen free (SPF) mouse models according to *Pseudomonas aeruginosa* infection are shown. More specifically, it can be shown that the survival rate of the SPF mouse model according to the intranasal infection of *Pseudomonas aeruginosa* was longer than the survival rate of the germ free mouse model. This may mean that the symbiotic microorganisms present in the respiratory tract of the SPF mouse model are factors determining host susceptibility to *Pseudomonas aeruginosa.* Accordingly, symbiotic microorganisms present in the respiratory tract may be used as a new therapeutic agent for preventing colonization and infection of *Pseudomonas aeruginosa.*

### Selection of Staphylococcus epidermidis strain used in various Examples of the present invention

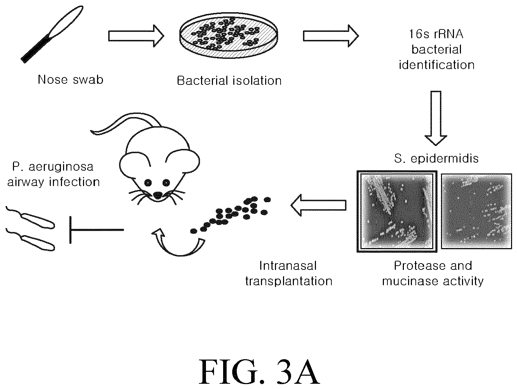

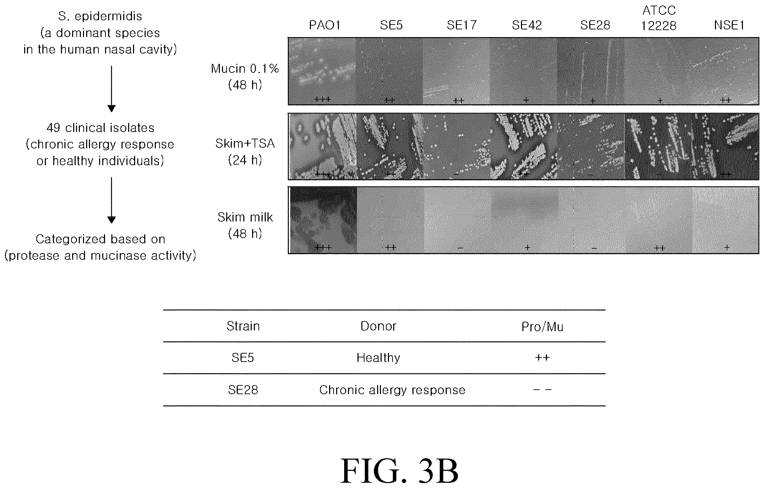

Hereinafter, a selection process for *Staphylococcus epidermidis* strains according to an embodiment of the present invention is described with reference to FIGS. 3A and 3B.

FIGS. 3A and 3B illustrate the selection process of *Staphylococcus epidermidis* strain according to an embodiment of the present invention.

First, referring to FIG. 3A, for the selection of *Staphylococcus epidermidis* strain according to an embodiment of the present invention, biological samples were collected from the nasal cavity of a chronic allergic individual and a healthy individual, and then the resident flora was cultured and isolated to perform 16s rRNA analysis, and among them, it was performed by selecting a specific strain with potential for colonization in the nasal cavity environment.

More specifically, referring to FIG. 3B, protease is a proteolytic enzyme, and the strain in which the expression of protease is activated can maintain energy metabolism even in a harsh environment, and mucinase is a hydrolysis catalytic enzyme, and the strain in which the expression of mucinase is activated can stably attach and proliferate in a harsh environment. Accordingly, the strain may improve the colonization ability through protease and mucinase.

Accordingly, *Staphylococcus epidermidis* was selected based on protease and mucinase, and then a *Staphylococcus epidermidis* strain SE5 according to an embodiment of the present invention expressing all of them was selected.

In other words, the *Staphylococcus epidermidis* strain SE5 according to an embodiment of the present invention has activity (+) for both protease and mucinase, so that it has superior colonization ability than other strains in various harsh environments including the nasal cavity.

Furthermore, the *Staphylococcus epidermidis* strain SE5 according to an embodiment of the present invention has activity (+) for both protease and mucinase, meaning that the strain is enhanced compared to the intrinsic activity for protease and mucinase.

Accordingly, SE5 was selected as a *Staphylococcus epidermidis* strain according to an embodiment of the present invention, and SE28 selected from chronic allergic reaction individuals was selected as its negative control. At this time, the isolated strain No. 5 having positive (+) activity for protease and mucinase was named SE5 for convenience of understanding of Examples herein but may refer to the same strain as *Staphylococcus epidermidis* PO01 specified in the deposited strain.

Through the above process, it was possible to obtain a specific *Staphylococcus epidermidis* species among the strains constituting the microbial community in the nasal cavity. At this time, the obtained specific *Staphylococcus epidermidis* strain may be used in various Examples in the present specification as a *Staphylococcus epidermidis* (Accession No.: KCTC13941BP) strain.

### Conferring host resistance to pathogenic bacteria of Staphylococcus epidermidis strain according to an embodiment of the present invention

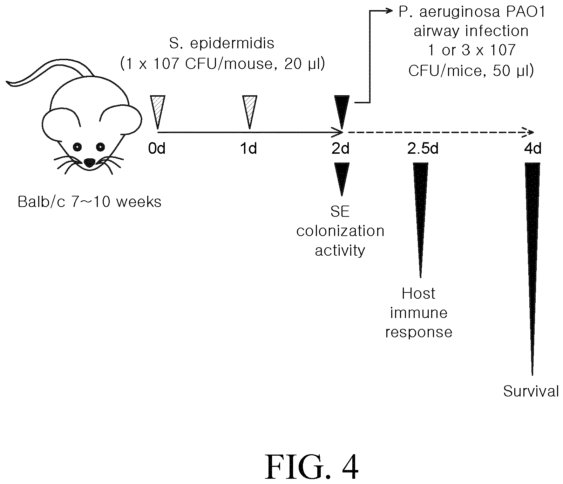

Hereinafter, the host resistance to pathogenic bacteria of the *Staphylococcus epidermidis* strain according to an embodiment of the present invention is described with reference to FIGS. 4 to 5C.

First, referring to FIG. 4, an experimental procedure for transplantation of a *Staphylococcus epidermidis* strain according to an embodiment of the present invention is illustrated.

The experimental procedure for transplantation of the *Staphylococcus epidermidis* strain according to an embodiment of the present invention was performed as follows: first, 1 × 10⁷ cfu/mice of *Staphylococcus epidermidis* strains (SE5 and SE28) was inoculated in the respiratory tract of a 7- to 10-week-old SPF mouse model (Balb/c) twice at an interval of 24 hours, and 24 hours later, 1 to 3 × 10⁷ cfu/mice of *Pseudomonas aeruginosa* (PAO1) was inoculated. Furthermore, on the day of inoculation with *Pseudomonas aeruginosa,* colonization activity for *Staphylococcus epidermidis* strains was measured. The host immune response was measured 12 hours after inoculation. The survival rate of each strain was measured 48 hours after *Pseudomonas aeruginosa* inoculation. The survival rate of the mouse model was measured 4 days after *Pseudomonas aeruginosa* inoculation.

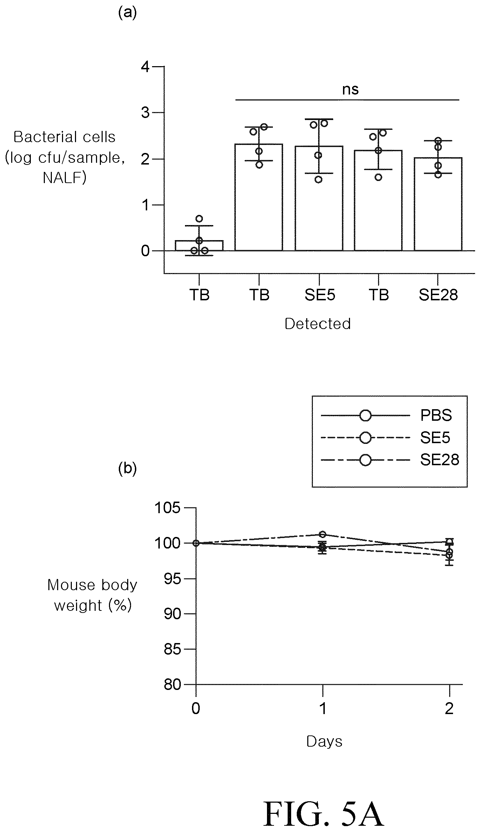

Accordingly, referring to FIG. 5A, colonization activity for the *Staphylococcus epidermidis* strain on the day of inoculation with *Pseudomonas aeruginosa* in the experimental procedure of FIG. 4 as described above is shown. First, referring to (a) of FIG. 5A, the number of bacteria in the individual inoculated with PBS was 0 to 1 log cfu/sample, indicating the lowest bacterial habitat rate. The number of bacteria in the individual inoculated with SE5 and SE28 was 2 to 3 log cfu/sample so that there was no difference between the two strains, and the number of bacteria did not differ from the number of total bacteria (TB) in each individual. Furthermore, the number of bacteria in the individual inoculated with SE5 and SE28 was shown to be 10² to 10³ times higher than the number of bacteria in the individual inoculated with PBS.

In other words, it may mean that the number of symbiotic microorganisms of an individual may be increased by transplantation, i.e., inoculation, of the *Staphylococcus epidermidis* strain according to an embodiment of the present invention.

Further, referring to (b) of FIG 5A, due to SE5 and SE28 inoculation, the body weight of the individual was not different from that of the individual inoculated with PBS, which means that SE5 and SE28 do not affect the individual.

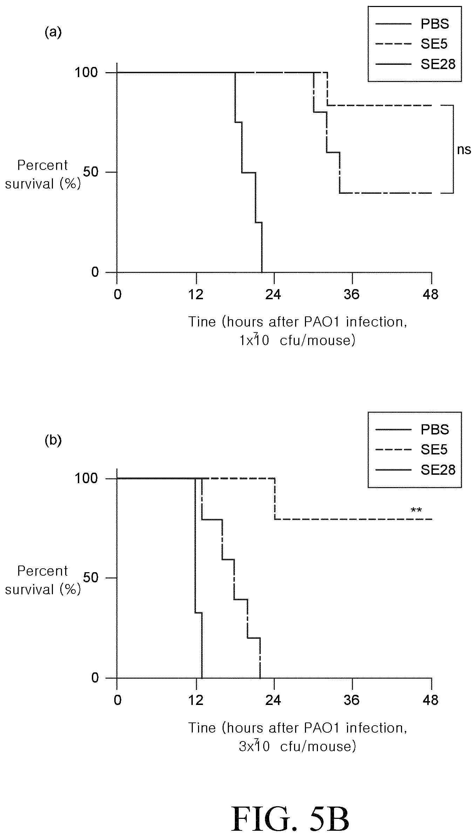

Referring to FIG. 5B, 2 days after *Pseudomonas aeruginosa* inoculation (or 48 hours later), the survival rate of the mouse model in the experimental procedure of FIG. 4 described above is shown.

First, referring to (a) of 5B, when 1 × 10⁷ cfu/mouse of *Pseudomonas aeruginosa* was inoculated, all the individuals inoculated with PBS died within 24 hours, and the survival rate of individuals inoculated with SE5 was about 80%, and the survival rate of individuals inoculated with SE28 was about 40%. That is, it was shown that the individual inoculated with SE5 has the highest survival rate.

Further, referring to (b) of 5B, when 3 × 10⁷ cfu/mouse of *Pseudomonas aeruginosa* was inoculated, all the individuals inoculated with PBS died within 13 hours, and the survival rate of individuals inoculated with SE5 was about 80%, and all individuals inoculated with SE28 died within 24 hours. That is, it was shown that the individual inoculated with SE5 has the highest survival rate, and the effect of conferring host resistance to pathogenic bacteria between the inoculated strains can be clearly exhibited as the concentration of *Pseudomonas aeruginosa* increases.

Therefore, the *Staphylococcus epidermidis* strain SE5 according to an embodiment of the present invention may improve the viability of the host against the pathogenic bacteria *Pseudomonas aeruginosa.*

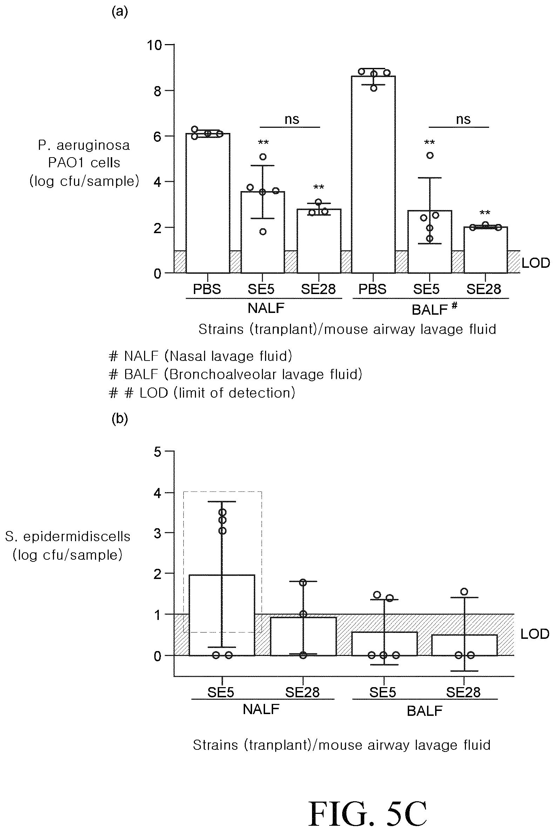

Furthermore, referring to FIG. 5C, the results for the survival rate of each strain are shown 48 hours after *Pseudomonas aeruginosa* inoculation in the experimental procedure of FIG. 4 described above. At this time, the survival rate of the strain was measured 48 hours after *Pseudomonas aeruginosa* inoculation in which the individual's nasal lavage fluid (NALF) and bronchoalveolar lavage fluid (BALF) were washed with physiological saline, and the number of strains for each washing solution was measured.

Referring to (a) of FIG. 5C, 48 hours after *Pseudomonas aeruginosa* inoculation, the number of *Pseudomonas aeruginosa* in the nasal cavity was not different between the individuals inoculated with SE5 and SE28 and was less than that of the individual inoculated with PBS. Likewise, the number of *Pseudomonas aeruginosa* in the alveolar was not different between the individuals inoculated with SE5 and SE28 and was less than that of the individuals inoculated with PBS. In other words, SE5, which is a *Staphylococcus epidermidis* strain according to an embodiment of the present invention was inoculated to reduce the survival rate or proliferation rate of pathogenic bacteria in the respiratory tract of an individual.

Meanwhile, referring to (b) of FIG. 5c, 48 hours after *Pseudomonas aeruginosa* inoculation, the number of *Staphylococcus epidermidis* strains of SE5 was more than that of SE28 in the nasal cavity, but there was no difference in the number of *Staphylococcus epidermidis* strains in the alveoli. In other words, SE5, a *Staphylococcus epidermidis* strain according to an embodiment of the present invention, may improve viability and proliferation in a nasal cavity environment that may be poorer in proliferation and viability, and this may result from enhanced intrinsic activity, i.e., protease and mucinase.

Accordingly, SE5, the *Staphylococcus epidermidis* strain according to an embodiment of the present invention may survive and proliferate in the respiratory tract for a long time, thereby increasing the duration of the defense mechanism effect against pathogenic bacteria. Furthermore, it may have high viability and proliferation in the nasal cavity to primarily improve the immune effect against pathogenic bacteria in the respiratory system of an individual.

According to the above results, the *Staphylococcus epidermidis* strain according to an embodiment of the present invention may confer resistance to pathogenic bacteria to the host, and thus have preventive and therapeutic effects against various diseases that can be caused by pathogenic bacteria.

### Confirmation of stability of Staphylococcus epidermidis strain according to an embodiment of the present invention

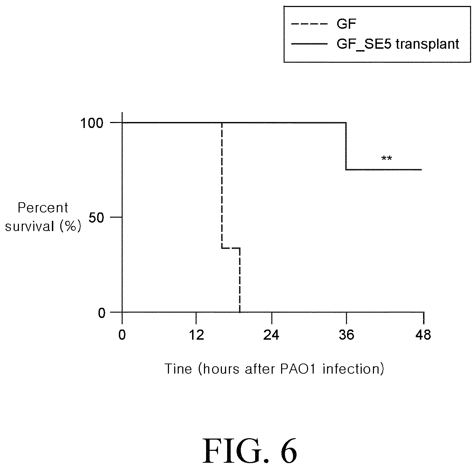

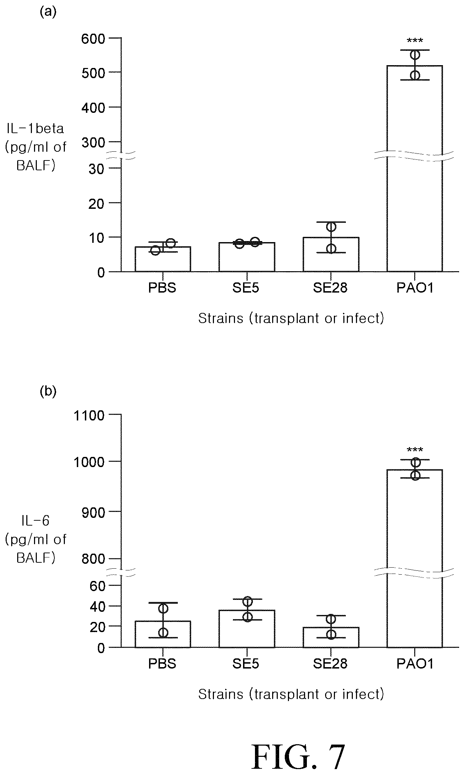

Hereinafter, the stability of the *Staphylococcus epidermidis* strain according to an embodiment of the present invention is described with reference to FIGS. 6 and 7.

First, referring to FIG. 6, the survival rate results of the germ free (GF) mouse model according to the transplantation of the *Staphylococcus epidermidis* strain according to an embodiment of the present invention are illustrated.

In the case of germ free mouse individuals inoculated with the *Staphylococcus epidermidis* strain SE5 according to an embodiment of the present invention, the survival rate was maintained at 80% until 48 hours of *Pseudomonas aeruginosa* inoculation, whereas all germ free mouse individuals not inoculated with SE5 died within 24 hours of *Pseudomonas aeruginosa* inoculation. That is, the *Staphylococcus epidermidis* strain according to an embodiment of the present invention does not affect the viability of germ free mice and may impart resistance to pathogenic bacteria to germ free mice.

Furthermore, referring to FIG. 7, the results for confirming the stability of the *Staphylococcus epidermidis* strain according to an embodiment of the present invention are illustrated. Here, the stability of the strain was confirmed through the secretion of cytokines IL-1 and IL-6 in the individual bronchoalveolar lavage fluid (BALF).

First, referring to FIG. 7(a), the concentrations of IL-1 bepa for the individuals inoculated with PBS, SE5 and SE28 were 8 to 12 pg/ml, which was similar without a statistical difference, and the concentration of IL-1 bepa for the individual inoculated with *Pseudomonas aeruginosa* was 500 to 600 pg/ml, which was significantly higher than those of the individuals inoculated with PBS, SE5 and SE28 described above.

Further, referring to FIG. 7(b), the concentrations of IL-6 for the individuals inoculated with PBS, SE5 and SE28 were 20 to 40 pg/ml, which was similar without statistical difference, and the concentration of IL-1 bepa for the individual inoculated with *Pseudomonas aeruginosa* was 900 to 1000 pg/ml, which was significantly higher than those of the individuals inoculated with PBS, SE5 and SE28 described above (p < 0.001).

Accordingly, it may mean that SE5, a *Staphylococcus epidermidis* strain according to an embodiment of the present invention, does not cause an immune response, unlike *Pseudomonas aeruginosa,* a pathogenic bacterium in an individual, and does not have harmful effects like PBS. In other words, SE5, a *Staphylococcus epidermidis* strain according to an embodiment of the present invention has stability without toxicity and harm to the individual and may coexist within the individual.

### Host immune-enhancing activity of Staphylococcus epidermidis strain according to an embodiment of the present invention

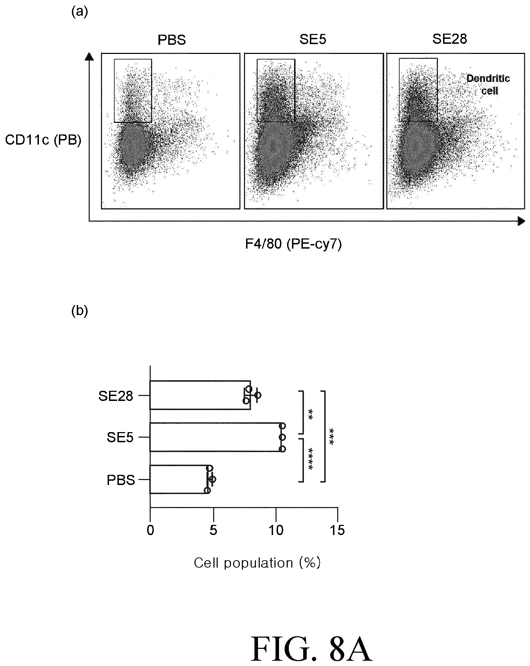

Hereinafter, the host immune-enhancing activity of the *Staphylococcus epidermidis* strain according to an embodiment of the present invention is described with reference to FIGS. 8A to 9B.

Referring to 8A to 8C, results for immune cell activity according to the transplantation of *Staphylococcus epidermidis* strain according to an embodiment of the present invention are illustrated. Here, immune cell activity was confirmed through flow cytometry after fluorescently staining each immune cell.

First, referring to FIG. 8A, the expression result of dendritic cells according to the transplantation of the *Staphylococcus epidermidis* strain according to an embodiment of the present invention is illustrated. The expression of dendritic cells in the individuals inoculated with SE5 was approximately 10% of the total immune cells, which was significantly higher than those of individuals inoculated with PBS and SE28 (p < 0.001, P < 0.05). Here, the number of dendritic cells of the individual inoculated with SE5, a *Staphylococcus epidermidis* strain according to an embodiment of the present invention was increased by 2 times or more than that of the individual inoculated with PBS, that is, the individual not inoculated with SE5, a *Staphylococcus epidermidis* strain according to an embodiment of the present invention.

Accordingly, SE5, a *Staphylococcus epidermidis* strain according to an embodiment of the present invention may improve the expression of dendritic cells, which are innate immune cells.

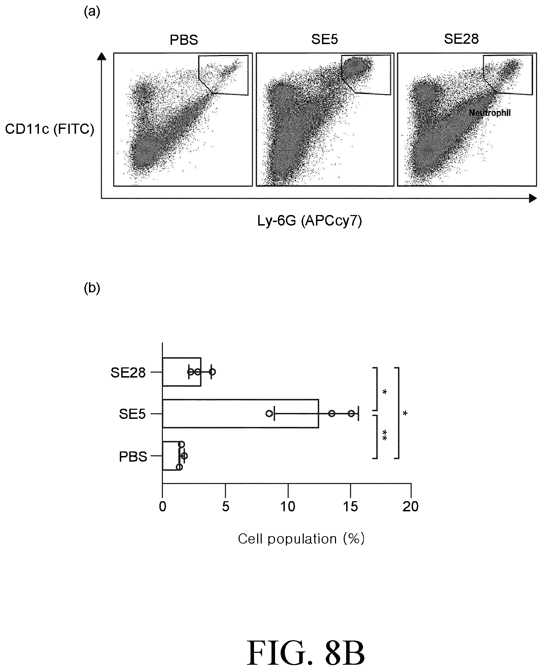

Referring to FIG. 8B, the expression result of neutrophils according to the transplantation of the *Staphylococcus epidermidis* strain according to an embodiment of the present invention is illustrated. The expression of neutrophils in the individuals inoculated with SE5 was approximately 13% of the total immune cells, which was significantly higher than those of individuals inoculated with PBS and SE28 (p < 0.001, P < 0.05). Here, the number of neutrophils of the individual inoculated with SE5, a *Staphylococcus epidermidis* strain according to an embodiment of the present invention was increased by 6 times or more than that of the individual inoculated with PBS, that is, the individual not inoculated with SE5, a *Staphylococcus epidermidis* strain according to an embodiment of the present invention.

Accordingly, SE5, a *Staphylococcus epidermidis* strain according to an embodiment of the present invention may improve the expression of neutrophils, which are innate immune cells.

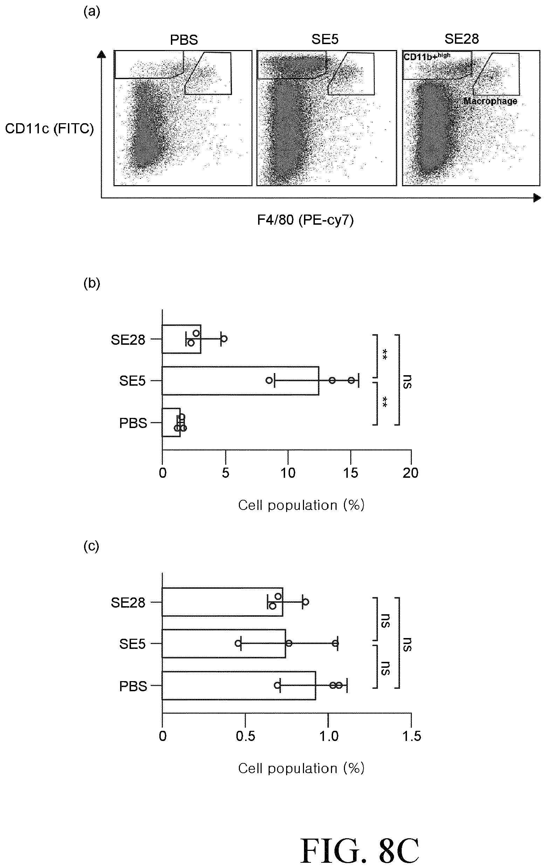

Referring to FIG. 8C, the expression result of monocytes according to the transplantation of the *Staphylococcus epidermidis* strain according to an embodiment of the present invention is illustrated. The expression of cells expressing CD11b(+), a marker of monocytes in the individuals inoculated with SE5, was approximately 13% of the total immune cells, which was significantly higher than those of individuals inoculated with PBS and SE28 (p < 0.001, P < 0.05). Here, the number of monocytes of the individual inoculated with SE5, a *Staphylococcus epidermidis* strain according to an embodiment of the present invention was increased by 12 times or more than that of the individual inoculated with PBS, that is, the individual not inoculated with SE5, a *Staphylococcus epidermidis* strain according to an embodiment of the present invention.

Accordingly, SE5, a *Staphylococcus epidermidis* strain according to an embodiment of the present invention may improve the expression of monocytes, which are innate immune cells.

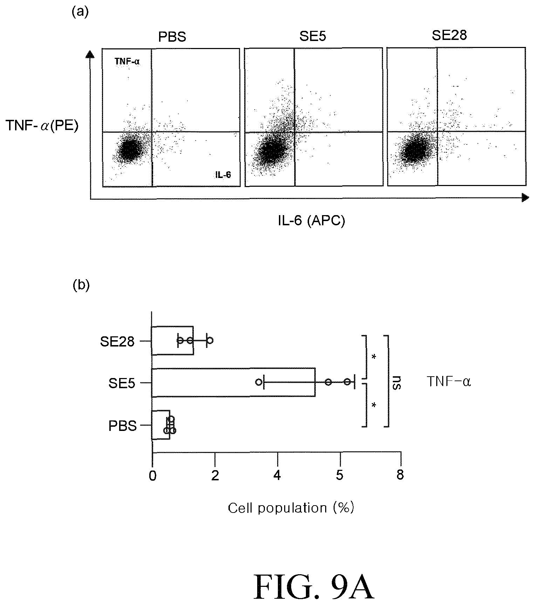

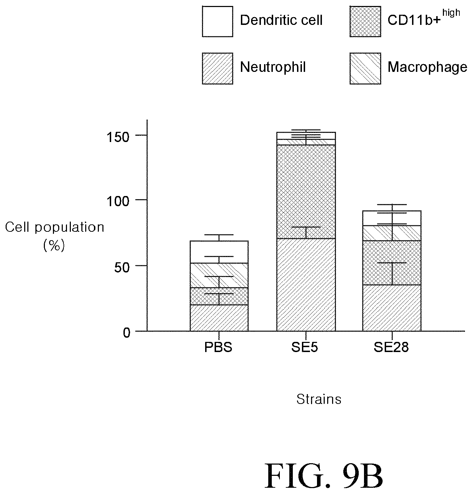

Furthermore, referring to FIGS. 9A and 9B, the results of cytokine secretion activity of immune cells according to the transplantation of *Staphylococcus epidermidis* strain according to an embodiment of the present invention are illustrated. Here, the cytokine was measured using TNF-α. TNF-α, tumor necrosis factor, is a mediator of the inflammatory response by the infectious bacteria and is a cytokine produced mainly in macrophages, but it can also be produced in various cells such as lymphocytes, NK cells, fibroblasts, mast cells, and keratinocytes as well as neutrophils, which plays a large role in the inflammatory response. Furthermore, such TNF-α can perform various functions such as anti-tumor activity, antibacterial activity, differentiation proliferation control and inflammation control.

Referring to Figure 9A, the cytokine expression results of immune cells according to the transplantation of *Staphylococcus epidermidis* strain according to an embodiment of the present invention is illustrated. First, referring to (a) of FIG. 9A, there was no difference in the expression of cytokines between the individuals inoculated with PBS and SE28. However, the expression of the cytokines of the individuals inoculated with SE5 had increased TNF-α in the second quadrant compared to the individuals inoculated with PBS and SE28.

More specifically, referring to (b) of FIG. 9A, the number of cells expressing TNF-α of the individual inoculated with SE25 was shown to account for about 5% of the total immune cells, which was significantly higher than those of the individuals inoculated with PBS and SE28 (p < 0.001, P < 0.05). Here, the number of cells expressing TNF-α of the individual inoculated with SE5, a *Staphylococcus epidermidis* strain according to an embodiment of the present invention, was increased by 5 times or more than that of the individual inoculated with PBS, that is, the individual not inoculated with SE5, a *Staphylococcus epidermidis* strain according to an embodiment of the present invention.

Accordingly, SE5, a *Staphylococcus epidermidis* strain according to an embodiment of the present invention may enhance the expression of cytokines in innate immune cells, thereby activating secondary acquired immune cells.

Furthermore, referring to FIG. 9B, the results for the ratio of innate immune cells to the cytokine-secreting cells of FIG. 9A are illustrated. In the case of individuals inoculated with PBS, the ratio of innate immune cells to cytokine-secreting cells was all similar. However, in the case of individuals inoculated with SE5, the ratio of innate immune cells to cytokine-secreting cells in monocyte (CD11b+ high) and neutrophils was shown to be the highest. In other words, SE5, a *Staphylococcus epidermidis* strain according to an embodiment of the present invention may activate cytokine secretion of monocytes (CD11b+ high) and neutrophils among innate immune cells the most.

According to the above results, the *Staphylococcus epidermidis* strain according to an embodiment of the present invention increases the number of innate immune cells of an individual and improves its cytokine secretion activity, thereby providing an improved immune-enhancing activity to the individual.

### Confirmation of antibacterial activity and effect of preventing and treating infectious diseases of Staphylococcus epidermidis strain according to an embodiment of the present invention

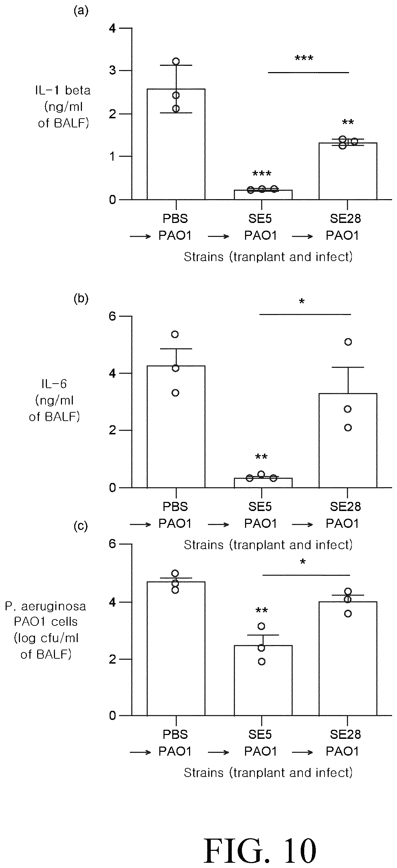

Hereinafter, the antibacterial activity and the effect of preventing and treating infectious diseases of the *Staphylococcus epidermidis* strain according to an embodiment of the present invention is described with reference to FIGS. 10 to 11

Referring to FIG. 10, the results for cytokine secretion and viability of the pathogenic strain according to infection with the pathogenic strain are illustrated. Here, the measurement of cytokine secretion and viability of the pathogenic strain was measured 12 hours after SE5 or SE28 inoculation and then infection with the pathogenic bacteria. Furthermore, cytokines were measured using IL-1 (beta) and IL-6, and IL-1 (interleukin-1) is a mediator of an individual's inflammatory response to infection or stimulation similar to TNF-α and can be generated from various types of cells such as monocytes, macrophages, keratinocytes, NK cells, T cells, B cells, endothelial cells, mast cells, neutrophils, fibroblasts, and nerve cells through stimulation of various production-inducing substances including microorganisms. Furthermore, IL-6 (interleukin-6) is a multifunctional mediator acting in both innate and acquired immunity and can be generated from lymphoid or non-lymphoid cells, that is, various types of cells such as monocytes, B cells, T cells, vascular endothelial cells, fibroblasts, and keratinocytes. When these cytokines are secreted in an appropriate amount, they may confer resistance to pathogenic bacteria, but when secreted in excess, cytokines may attack normal cells of an individual to cause cytokine storm, a secondary infection, which modifies the DNA of normal cells.

First, referring to (a) and (b) of FIG 10, the secretion amount of IL-1 (beta) according to the infection of the pathogenic bacteria in the individual inoculated with SE5 was about 0.3 ng/ml, which was the significantly smallest (p < 0.001), likewise, the secretion amount of IL-6 in the individual inoculated with SE5 was about 0.3 ng/ml, which was the significantly smallest (p < 0.001).

Furthermore, referring to FIG. 10(c), the survival rate of pathogenic bacteria in the individual inoculated with SE5 was about 2.3 log cfu/ml, which was the significantly smallest (p < 0.001).

Accordingly, it may mean that SE5, a *Staphylococcus epidermidis* strain according to an embodiment of the present invention, may not cause a cytokine storm, secrete a minimum of cytokines, and most effectively reduce the survival rate of pathogenic bacteria. After all, SE5, a *Staphylococcus epidermidis* strain according to an embodiment of the present invention may reduce side effects on the immune response at a minimum.

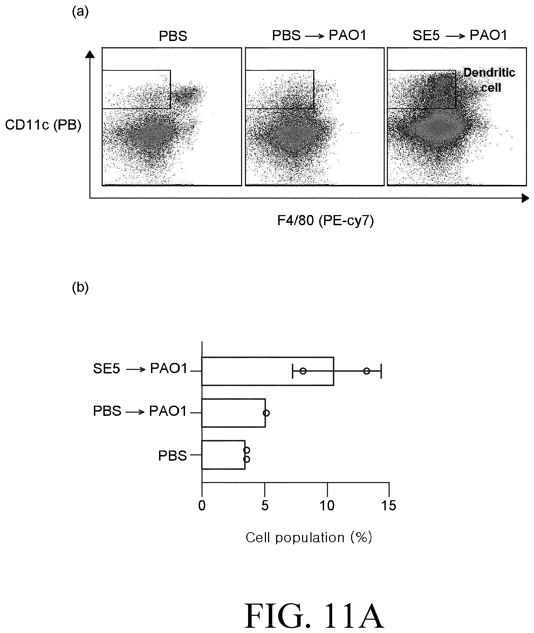

Furthermore, referring to FIG. 11A, the expression results of dendritic cells according to the transplantation of *Staphylococcus epidermidis* strain according to an embodiment of the present invention after infection with pathogenic bacteria are illustrated. The expression of dendritic cells in the individuals inoculated with SE5 was approximately 11% of the total immune cells, which was the highest. Here, the number of dendritic cells of the individual inoculated with SE5, a *Staphylococcus epidermidis* strain according to an embodiment of the present invention was increased by 2 times or more than that of the individual inoculated with PBS, that is, the individual not inoculated with SE5, a *Staphylococcus epidermidis* strain according to an embodiment of the present invention.

Accordingly, SE5, a *Staphylococcus epidermidis* strain according to an embodiment of the present invention may improve the expression of dendritic cells, which are innate immune cells.

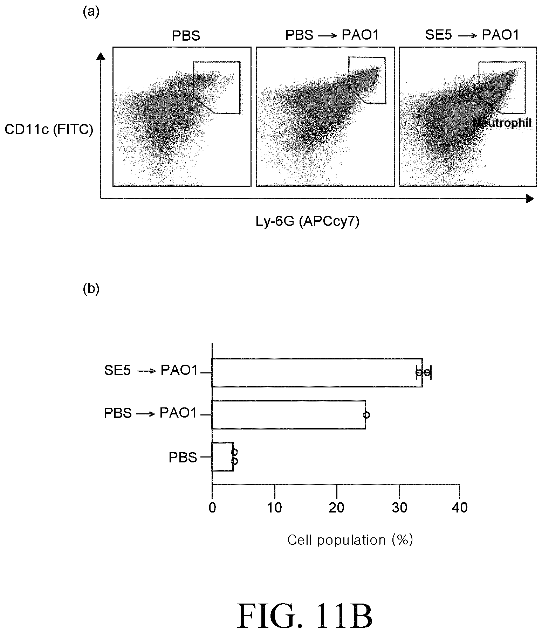

Furthermore, referring to FIG. 11B, the expression results of neutrophils according to the transplantation of *Staphylococcus epidermidis* strain according to an embodiment of the present invention after infection with pathogenic bacteria are illustrated. The expression of neutrophils in the individuals inoculated with SE5 was approximately 35% of the total immune cells, which was the highest. Here, the number of neutrophils of the individual inoculated with SE5, a *Staphylococcus epidermidis* strain according to an embodiment of the present invention was increased by 1.5 times or more than that of the individual inoculated with PBS, that is, the individual not inoculated with SE5, a *Staphylococcus epidermidis* strain according to an embodiment of the present invention. Furthermore, the expression rate of the expressed neutrophils after infection with pathogenic bacteria was higher than the expression rate in FIG. 8B described above.

In other words, SE5, a *Staphylococcus epidermidis* strain according to an embodiment of the present invention, primarily increases neutrophils, which are innate immune cells, to an individual, thereby warming up a preventive effect against pathogenic bacteria such as vaccines and external stimuli. Accordingly, it is possible to secondarily boost the immune response to the pathogenic bacteria.

Accordingly, SE5, a *Staphylococcus epidermidis* strain according to an embodiment of the present invention may improve the expression of neutrophils, which are innate immune cells, thereby further enhancing the secondary acquired immune response, and thus the individual's immunity reaction may be improved more efficiently.

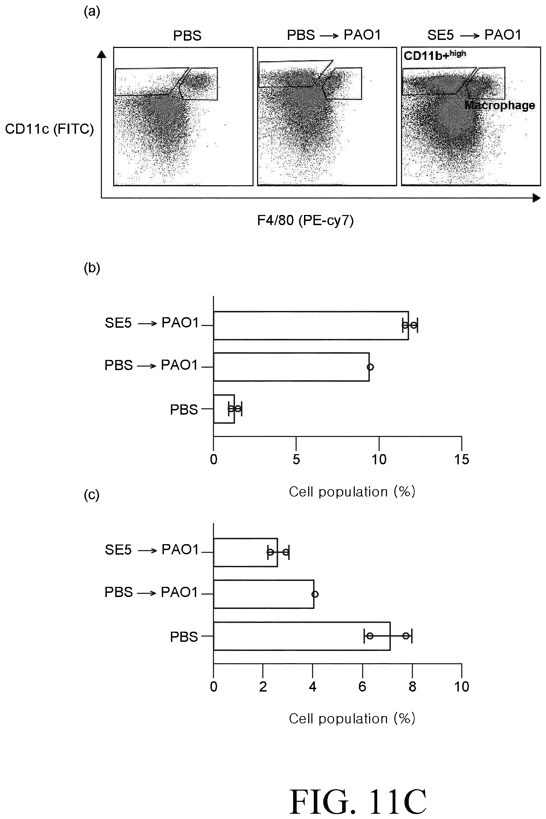

Furthermore, referring to FIG. 11C, the expression result of monocytes according to the transplantation of *Staphylococcus epidermidis* strain according to an embodiment of the present invention after infection with pathogenic bacteria is illustrated. The expression of cells expressing CD11b(+), a marker of monocytes in the individual inoculated with SE5 was about 13% of the total immune cells, which was the highest. Here, the number of monocytes of the individual inoculated with SE5, a *Staphylococcus epidermidis* strain according to an embodiment of the present invention was increased by 1.4 times or more than that of the individual inoculated with PBS, that is, the individual not inoculated with SE5, a *Staphylococcus epidermidis* strain according to an embodiment of the present invention. Furthermore, the expression rate of cells expressing CD11b(+), a marker of the expressed monocytes after infection with pathogenic bacteria was higher than the expression rate in FIG. 8C described above.

In other words, SE5, a *Staphylococcus epidermidis* strain according to an embodiment of the present invention, primarily increases cells expressing CD11b(+), which are innate immune cells, a marker of monocytes, to an individual, thereby warming up a preventive effect against pathogenic bacteria such as vaccines and external stimuli. Accordingly, it is possible to secondarily boost the immune response to the pathogenic bacteria.

Accordingly, SE5, a *Staphylococcus epidermidis* strain according to an embodiment of the present invention may improve the expression of monocytes, which are innate immune cells, thereby further enhancing the secondary acquired immune response, and thus the immune response of the individual may be improved effectively.

Each feature of the various embodiments of the present invention can be partially or wholly connected or combined with each other and technically variously coupled and operated as will be fully understood by those skilled in the art. The respective Examples are implemented independently or in association with respect to each other.

Although the Examples of the present invention have been described in more detail with reference to the accompanying drawings, the present invention is not necessarily limited to these Examples, and various modifications may be made within the scope without departing from the technical spirit of the present invention. Accordingly, the Examples disclosed in the present invention are not intended to limit the technical spirit of the present invention, but to explain the present invention, and the scope of the technical spirit of the present invention is not limited by these Examples. Therefore, it should be understood that the Examples described above are illustrative and not restrictive in all respects. The protection scope of the present invention should be construed by the following claims, and all technical ideas within the scope equivalent thereto should be construed as being included in the scope of the present invention.

### [National R&D project supporting the present invention]

[Project unique number] 1711084367
[Project number] 017R1A2A2A05019987
[Name of ministry] Ministry of Science and ICT
[Name of project management (specialized) institution] National Research Foundation of Korea
[Name of research project] Personal Basic Research (Ministry of Science and ICT) (R&D)
[Title of research project] Understanding the mechanism of interaction between respiratory and intestinal pathogenic microorganisms and the host
[Contribution rate] 1/3
[Name of project performance institution] Yonsei University
[Research period] March 1, 2019 to February 29, 2020
[National R&D project supporting the present invention]
[Project unique number] 1545018734
[Project number] 918003042SB010
[Name of ministry] Ministry of agriculture, food and rural affairs
[Name of project management (specialized) institution] Institute of planning and evaluation for technology in food, agriculture and forestry
[Name of research project] Multi-ministerial genome project to foster a new post-genomic industry (R&D) (Ministry of agriculture, food and rural affairs) (R&D)

### (Ministry of Agriculture and Forestry)

[Title of research project] Development of probiotic strains that inhibit infection and relieve intestinal inflammation
[Contribution rate] 1/3
[Name of project performance institution] Yonsei University Industry-Academic Cooperation Foundation
[Research period] January 1, 2019 to December 31, 2019
[National R&D project supporting the present invention]
[Project unique number] 1345293841
[Project number] 2017R1D1A1B03031537
[Name of ministry] Ministry of education
[Name of project management (specialized) institution] National Research Foundation of Korea
[Name of research project] Personal Basic Research (Ministry of education) (R&D)
[Title of research project] Development of antibiotic-independent *Pseudomonas aeruginosa* infection control strategy
[Contribution rate] 1/3
[Name of project performance institution] Yonsei University
[Research period] March 1, 2019 to February 29, 2020

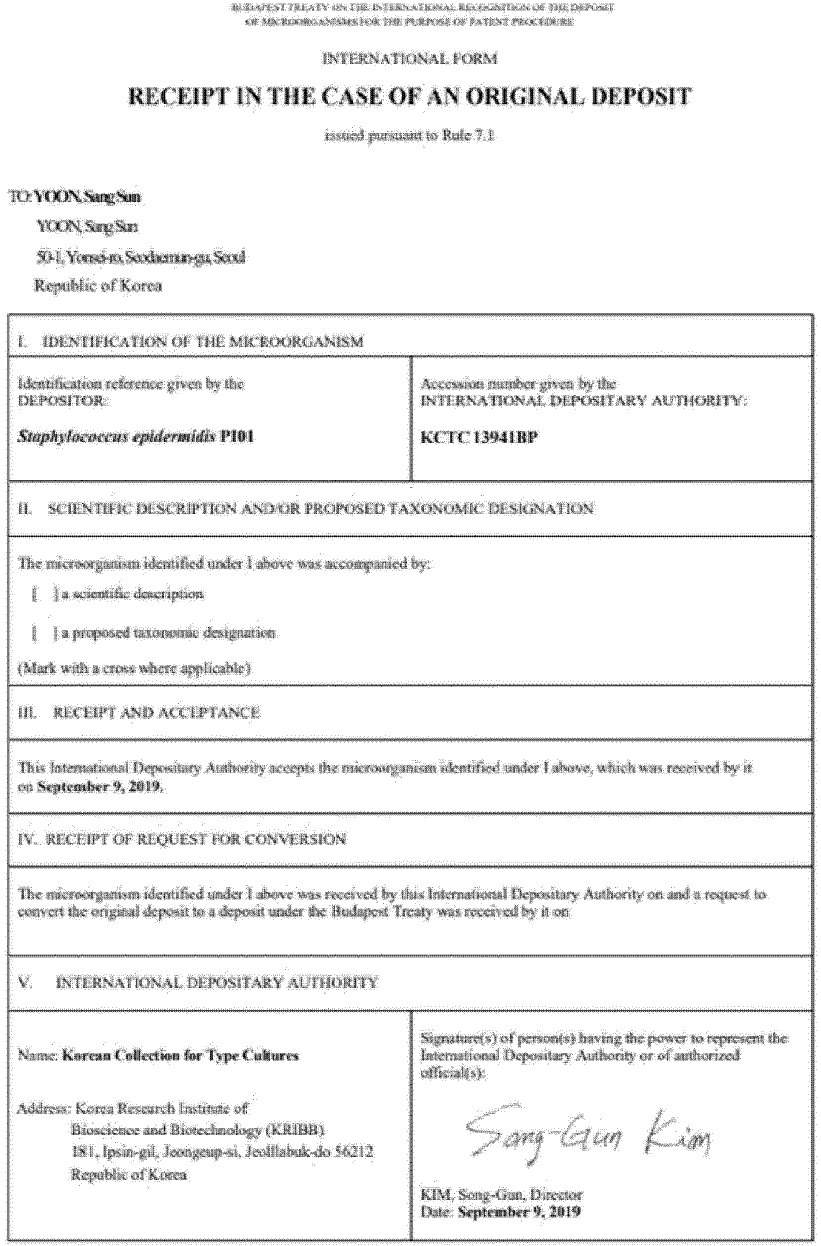
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: YOON, Sang Sun
YOON, Sang Sun
50-1, Yonsei-ro, Seodaemun-gu, Seoul
Republic of Korea

I. IDENTIFICATION OF THE MICROORGANISM
Identification reference given by the DEPOSITOR: | Accession number given by the INTERNATIONAL DEPOSITARY AUTHORITY:
Staphylococcus epidermidis P101 | KCTC 13941BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it on September 9, 2019.
IV. RECEIPT OF REQUEST FOR CONVERSION
The microorganism identified under I above was received by this International Depositary Authority on and a request to convert the original deposit to a deposit under the Budapest Treaty was received by it on
V. INTERNATIONAL DEPOSITARY AUTHORITY
Name: Korean Collection for Type Cultures | Signature(s) of person(s) having the power to represent the International Depositary Authority or of authorized official(s):
Address: Korea Research Institute of
Bioscience and Biotechnology (KRIBB)
181, Ipsin-gil, Jeongeup-si, Jeollabuk-do 56212
Republic of Korea |
KIM, Song-Gun, Director
Date: September 9, 2019

## Claims

1. A *Staphylococcus epidermidis* strain deposited with accession number KCTC13941BP and having an immune-enhancing activity.

2. The *Staphylococcus epidermidis* strain of claim 1, wherein the immune-enhancing activity is to increase innate immune cells expressing TNF-α.

3. The *Staphylococcus epidermidis* strain of claim 2, wherein the innate immune cells include at least one selected from the group consisting of dendritic cells, monocytes, neutrophils, eosinophils, basophils, macrophages, and natural killer cells.

4. The *Staphylococcus epidermidis* strain of claim 3, wherein the number of dendritic cells is increased by 2 times or more than that of an individual not treated with the *Staphylococcus epidermidis* strain.

5. The *Staphylococcus epidermidis* strain of claim 3, wherein the number of monocytes is increased by 12 times or more than that of an individual not treated with the *Staphylococcus epidermidis* strain.

6. The *Staphylococcus epidermidis* strain of claim 3, wherein the monocytes are CD11b+^{high} cells.

7. The *Staphylococcus epidermidis* strain of claim 3, wherein the number of neutrophils is increased by 6 times or more than that of an individual not treated with the *Staphylococcus epidermidis* strain.

8. The *Staphylococcus epidermidis* strain of claim 2, wherein the number of innate immune cells is increased by 2 times or more than that of an individual not treated with the *Staphylococcus epidermidis* strain.

9. The *Staphylococcus epidermidis* strain of claim 1, wherein the strain exhibits antibacterial activity against a pathogenic bacterium.

10. The *Staphylococcus epidermidis* strain of claim 9, wherein the pathogenic bacterium is *Pseudomonas aeruginosa.*

11. The *Staphylococcus epidermidis* strain of claim 10, wherein the number of *Pseudomonas aeruginosa* is 100 times or less than that of an individual not treated with the *Staphylococcus epidermidis* strain.

12. The *Staphylococcus epidermidis* strain of claim 9, wherein the pathogenic bacteria are resistant to at least one selected from the group consisting of a carbapenem-based antibacterial agent, beta-lactam-based antibacterial agent, penicillin-based antibacterial agent, cephalosporins-based antibacterial agent, and monobactams-based antibacterial agent.

13. The *Staphylococcus epidermidis* strain of claim 1, wherein the *Staphylococcus epidermidis* strain is enhanced compared to intrinsic activity for proteases and mucinases.

14. A pharmaceutical composition for preventing or treating infectious diseases, the composition comprising the strain of any one of claims 1 to 13 or a suspension of the strain as an active ingredient.

15. The pharmaceutical composition for preventing or treating infectious diseases of claim 14, wherein the infectious disease includes at least one selected from the group consisting of tuberculosis, pneumonia, urinary tract infection, wound infection, encephalomeningitis, osteomyelitis, wound infection, entophthalmia, endophthalmitis, liver abscess, pharyngitis, diarrhea, sepsis, sinusitis, rhinitis, otitis media, bacteremia, endocarditis, cholecystitis, and parotitis.

16. The pharmaceutical composition for preventing or treating infectious diseases of claim 14, wherein the pharmaceutical composition is an external preparation or a spray.

17. A composition for enhancing immunity, the composition comprising the strain of any one of claims 1 to 13 or a suspension of the strain as an active ingredient.
